Europäisches Patentamt

European Patent Office

Office européen des brevets

(11) Publication number: **0 265 785**
**A2**

# (12) EUROPEAN PATENT APPLICATION

(21) Application number: **87115085.0**

(22) Date of filing: **15.10.87**

(51) Int. Cl.⁴ **C12N 15/00** , **A61K 39/21** ,
**C12N 7/00** , **G01N 33/569** ,
**C12P 21/00** , **C07K 1/14**

(30) Priority: **16.10.86 US 920197**

(43) Date of publication of application:
**04.05.88 Bulletin 88/18**

(84) Designated Contracting States:
**AT BE CH DE ES FR GB GR IT LI LU NL SE**

(71) Applicant: **Microgenesys, Inc.**
**400 Frontage Road**
**West Haven New Haven Connecticut**
**06516(US)**

(72) Inventor: **Cochran, Mark A.**
**415 Brooksvale Avenue**
**Hamden Connecticut 06518(US)**
Inventor: **Smith, Gale E.**
**125 Michael Drive**
**Guilford§Connecticut 06437(US)**
Inventor: **Volvovitz, Franklin**
**123 York Street Apt. 18E**
**Crown Court New Heaven Connecticut**
**06516(US)**

(74) Representative: **Vossius & Partner**
**Siebertstrasse 4 P.O. Box 86 07 67**
**D-8000 München 86(DE)**

(54) **Polypeptides derived from the envelope gene of human immunodeficiency virus in recombinant baculovirus infected insect cells.**

(57) The baculovirus-insect cell vector system is used as a high efficiency eukaryotic cloning and expression method for the production of recombinant proteins. A major application of this system is also in the development of rDNA vaccines for a variety of important human and animal diseases. This system is used to express a variety of candidate vaccine immunogens derived from the envelope gene of Human Immunodeficiency Virus (HIV). The envelope protein of HIV is the most promising candidate in the development of vaccines and diagnostics for AIDS since it has been shown that the envelope glycoprotein represents the major target antigen of AIDS virus. Recombinant baculoviruses have been constructed which express a variety of domains of the envelope gene (env), including gp 160 and gp 120. Expression of the recombinant HIV baculoviruses in insect cells results in the production of glycoproteins which are reactive with HIV-specific serum. HIV antigens produced from some of these recombinants have been successfully employed in a variety of immunodiagnostic assays including ELISA, Western Blotting and radioimmunoprecipitation.

# POLYPEPTIDES DERIVED FROM THE ENVELOPE GENE OF HUMAN IMMUNODEFICIENCY VIRUS IN RECOMBINANT BACULOVIRUS INFECTED INSECT CELLS

Acquired immune deficiency syndrome (AIDS) is a viral disease of major global importance. The causative agent of the disease is a retrovirus called Human Immunodeficiency Virus (HIV), variously termed lymphadenopathy virus (LAV) (Barre-Sinoussi et al 1983), human T cell leukemia virus type III (HTLV-III) (Popovic et al. 1984), or AIDS-related virus (ARV) (Levy et al. 1984). The structure and gene organization of several AIDS viruses has been elucidated from the complete nucleotide sequence of molecular clones and direct sequencing of viral proteins.

The envelope protein is the most promising candidate in the development of an AIDS vaccine and diagnostic (Francis et al. 1985). Antibodies against envelope glycoprotein are commonly detected in sera from AIDS patients (Robey, et al. 1985). Furthermore, the envelope glycoprotein represents the major target antigen of AIDS virus (Barin et al. 1985).

The production of an effective vaccine for AIDS depends on the ability to manufacture large quantities of a safe antigen that stimulates protective immunity when it is injected into human beings. Recombinant DNA technology presents the best option to antigen production because of its perceived ability to produce large quantities of safe and economical immunogens. The choice of which recombinant systems (bacterial, yeast, or other eukaryotic cells) to use will involve consideration of the following concerns:

the envelope glycoprotein of HIV is specifically processed by glycosylation and cleavage. The recombinant system should be capable of processing the gene product in a desirable manner.

bacterial and yeast cells do not effectively glycosylate their expression proteins.

while mammalian cells appear to be appropriate expression vectors, they have the disadvantage of containing many undesirable immunoreactive antigens and furthermore, present the risk of harboring adventitious agents.

The baculovirus expression system presents an attractive and viable opportunity for the production of an effective HIV envelope immunogen.

Baculoviruses can be used as high efficiency eukaryotic cloning and expression vectors for the production of recombinant proteins in cultured insect cells.

Baculoviruses have more than the necessary requirements for use as eukaryotic cloning and expression vectors. Baculoviruses are safe by virtue of their narrow host range which is restricted to arthropods; they are capable of accommodating very large amounts of exogenous DNA; their cell culture systems are safe, non-transformed, and efficient; and they possess the highly efficient polyhedrin promoter, which is more active than any other known promoter in virus-infected eukaryotic cells.

The baculovirus system also offers strong advantages in the production of polypeptides for use in diagnostic procedures. Many humans and animals generally possess antibodies which react with bacterial, yeast and heterologous histocompatibility antigens. The presence of these antibodies diminishes the reliability of diagnostic procedures due to false positive reactions with contaminating bacterial, yeast and mammalian proteins found in preparations produced in the respective expression systems. Humans and animals most likely do not have prior history of immunlogic exposure to lepidopteran cell or baculovirus antigens. The lepidopteran cells and baculovirus employed in production of recombinant proteins in this system are therefore more likely not to have the problem of contaminating antigens that are recognized by antibody normally present in humans or animals. Therefore, contaminants of lepidopteran cell or baculovirus origin are not likely to lead to false positive reactions in diagnostic procedures employing recombinant antigens produced in this system.

The subject matter of the invention is an AIDS virus env protein expressed from a recombinant insect virus, e.g. a recombinant insect baculovirus. Specific examples are AIDS virus env gp160 protein, AIDS virus env gp120 protein, and AIDS virus env gp41 protein. Included are also immunogenic fragments of the AIDS virus env protein.

A further subject matter of the invention is a recombinant insect baculovirus having incorporated therein the AIDS virus env protein gene for expression of the AIDS virus env protein in an insect cell. Said virus may have incorporated therein a gene for an immunogenic fragment of the AIDS virus env protein, the gene for AIDS virus env gp160 protein, the gene for AIDS virus env gp120 protein, or the gene for AIDS virus env gp41 protein.

The AIDS virus env protein can be incorporated in or fixed onto a substrate or carrier, which may be a film, a transparent substrate or carrier, or a nitrocellulose film or membrane. Also the immunogenic fragment of the the AIDS virus env protein may be incorporated in or fixed onto a solid substrate or carrier.

A further subject matter of the invention is· a vaccine against AIDS virus comprising an immunogenic fragment of AIDS virus env protein. The vaccine may comprise AIDS virus env gp160 protein. AIDS virus env gp120 protein, or AIDS virus env gp41 protein.

A diagnostic composition useful for the detection of AIDS antibody comprises an immunogenic fragment of AIDS virus env protein and a liquid carrier therefor. The method of detecting AIDS virus antibody comprises contacting said AIDS virus antibody with AIDS env protein expressed from a recombinant insect virus and determining any fixing or reaction between said AIDS virus antibody and said protein. Preferably said AIDS virus env protein is labeled so as to detect the fixing or reaction of said protein with said AIDS virus antibody.

Subject matters of the invention include:

AIDS virus env protein expressed in recombinant infected cells;

AIDS virus env protein expressed in recombinant infected insect cells;

AIDS virus env proteins, envelope and core expressed in insect cells;

AIDS virus gag protein expressed from a recombinant insect virus; and

AIDS virus pol protein expressed from a recombinant insect virus.

A further subject matter of the invention is a method of producing AIDS virus env protein which comprises inserting the AIDS virus env protein gene or the AIDS virus envelope gene (env) into the DNA of an insect virus, infecting insect cells or insects with the resulting recombinant virus and culturing the resulting infected insect or insect cells to express or produce the AIDS virus env protein. Said insect virus is preferably a baculovirus.

Another subject matter of the invention is a method of constructing a recombinant baculovirus expression vector capable of expressing a selected AIDS virus gene or portion thereof in a host insect cell, comprising:

(a) preparing a recombinant insertion vector by inserting a portion of a baculovirus genome into a cloning vehicle and thereafter inserting a selected AIDS virus gene or portion thereof into the modified insertion vector such that the selected AIDS virus gene or portion thereof is expressed under the control of a baculovirus promoter,

(b) transferring the above modified AIDS virus gene or portion thereof inot a baculovirus expression vector by mixing the above modified insertion vector with baculovirus DNA, transfecting suitable insect cells, and isolating recombinant viruses that contain the selected AIDS gene or portion thereof.

In this method the selected AIDS virus gene or portion thereof is preferably the envgene or portion thereof, the gag gene or portion thereof, or the pol gene or portion thereof. The baculovirus promoter is preferably the polyhedrin gene promoter and the baculovirus is preferably Autographa californica Nuclear Polyhedrosis Virus. The selected AIDS virus protein gene or portion thereof are from the AIDS env gene and are most preferably in the insertion vectors, A, B, C, D, E, F, G, H, I, J, K, L, A-1, or B-1 as described and illustrated with reference to the accompanying drawings.

A further subject matter of the invention is a method for synthesizing a selected AIDS virus polypeptide which comprises infecting a susceptible host insect cell with a recombinant baculovirus vector wherein the baculovirus vector contains one or more AIDS virus protein genes or portions thereof and culturing the infected cell to express the AIDS virus polypeptide.

In this method the susceptible insect cell is preferably derived from the insect species Spodoptera frugiperda. The AIDS gene or portion thereof is preferably the env gene or portion thereof, the gag gene or portion thereof, or the pol gene or portion thereof. The AIDS virus genes or portions thereof are preferably expressed under the control of a baculovirus promoter, e.g. the polyhedrin gene promoter. The baculovirus is preferably Autographa californica Nuclear Polyhedrosis Virus. The selected AIDS virus protein gene or portion thereof are from the AIDS env gene and are most preferably in the insertion vectors A, B, C, D, E, F, G, H, I, J, K, L, A-1, or B-1 as described and illustrated with reference to the accompanying drawings.

Still a further subject matter of the invention is a method for the purification of AIDS env gene polypeptides comprising subjecting said polypeptides to lentil lectin affinity chromatography to isolate glycoproteins, followed by size tractionation of the glycoproteins using molecular sieve chromatography.

Still another subject matter of the invention are AIDS polypeptides or portions thereof produced in insect cells in accordance with the methods mentioned above, incorporated in or fixed to a solid substrate. The solid substrate may be made of polystyrene molded into a tray containing multiple wells. It may also be a filter or film.

Still another subject matter of the invention is a method for detection and mesuring of human AIDS which comprises contacting said antibody with the recombinant AIDS virus protein of the invention. The recombinant AIDS virus polypeptides or protein or portions thereof which are used in this method are preferably produced in accordance with the methods mentioned above. The recombinant AIDS protein used is preferably fixed to a solid substrate. Said contacting includes preferably an enzyme-linked solid-phase immuno-absorbent assay (ELISA) or Western Blot analysis.

Still another subject matter of the invention is a vaccine against AIDS disease comprising the AIDS virus env protein of the invention and a physiological carrier therefor.

In one embodiment of this invention the baculovirus Autographa californica nuclear polyhedrosis virus (AcMNPV) was used. The promoter sequence used for expression is that of the polyhedrin (occ) gene derived from this virus. AcMNPV and recombinant virus stocks were maintained in Spodoptera frugiperda - (fall armyworm) cells. The recombinant was constructed such that the Pn gene was deleted which renders the recombinant virus occ⁻ This allows for easy identification of recombinant viruses simply by plaque morphology. Once isolated a recombinant baculovirus can be used to infect any permissive or semi-permissive cell population maintained as a cultured line or as part of a whole insect.

Construction of Insertion Vectors

Cloning and expression of foreign protein coding sequence in a baculovirus vector requires that the coding sequence be aligned with the polyhedrin promoter and upstream sequences and on the other side with truncated polyhedrin coding sequences such that homologous recombination with the baculovirus genome results in transfer of the foreign coding sequence aligned with the polyhedrin promoter and an inactive polyhedrin gene.

Accordingly, a variety of insertion vectors were designed for use in AIDS env gene constructions. Each insertion vector described below was designed to supply the ATG translational initiating codon. Insertion of foreign sequences into these vectors must be engineered such that the translational frame established by the initiating codon is maintained correctly through the foreign sequences.

Details of the practices of this invention are set forth hereinbelow with reference to the accompanying drawings:

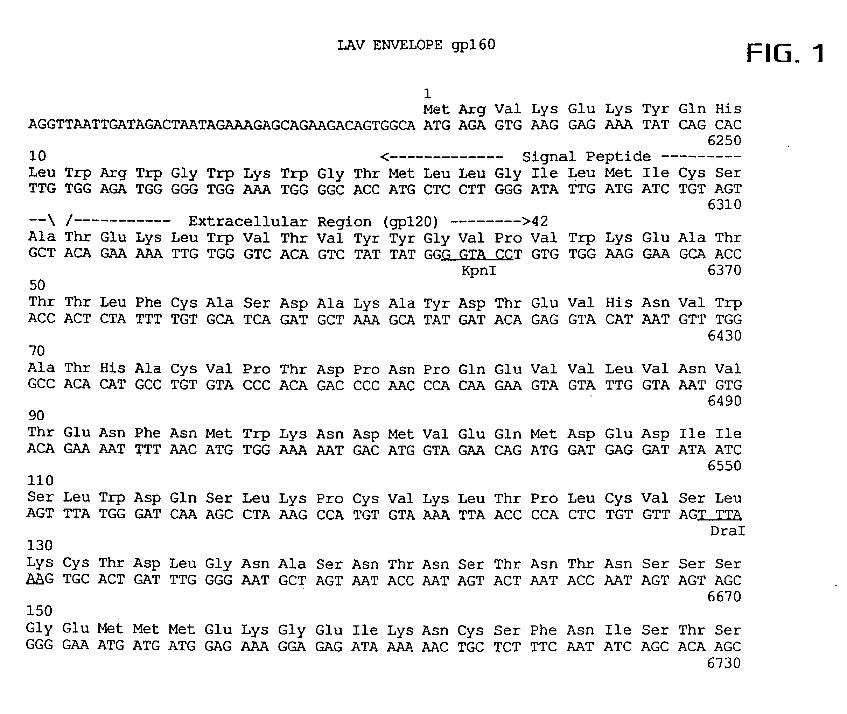

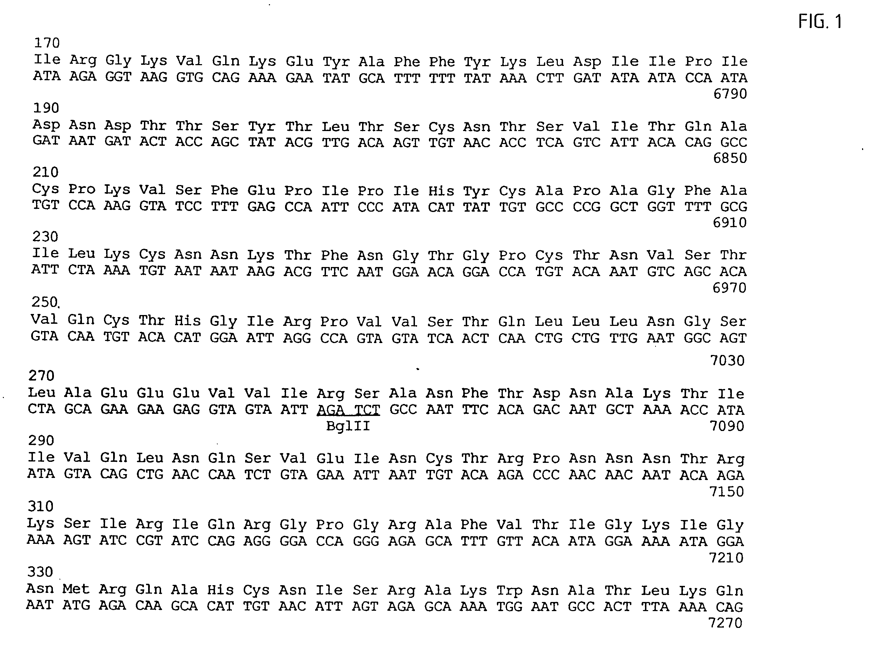

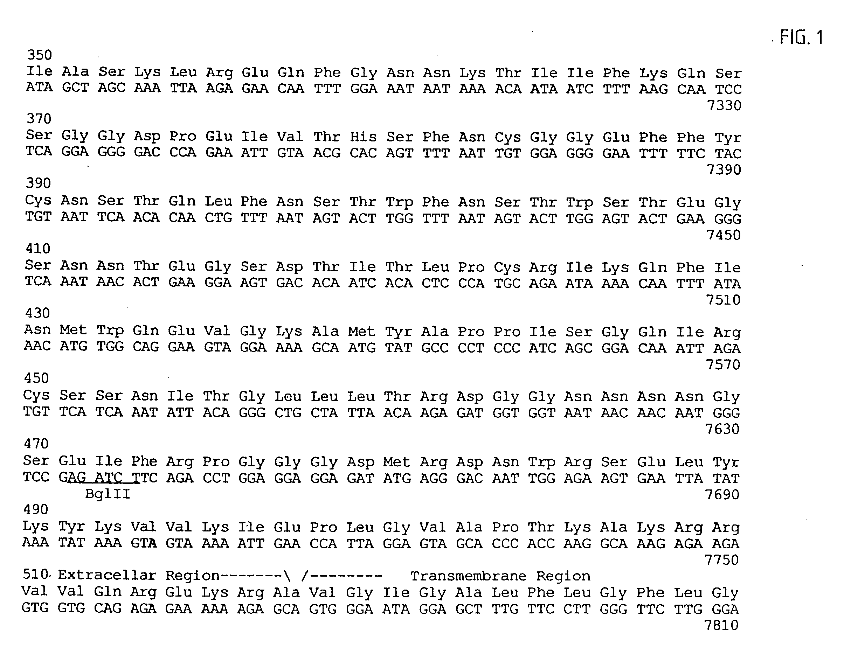

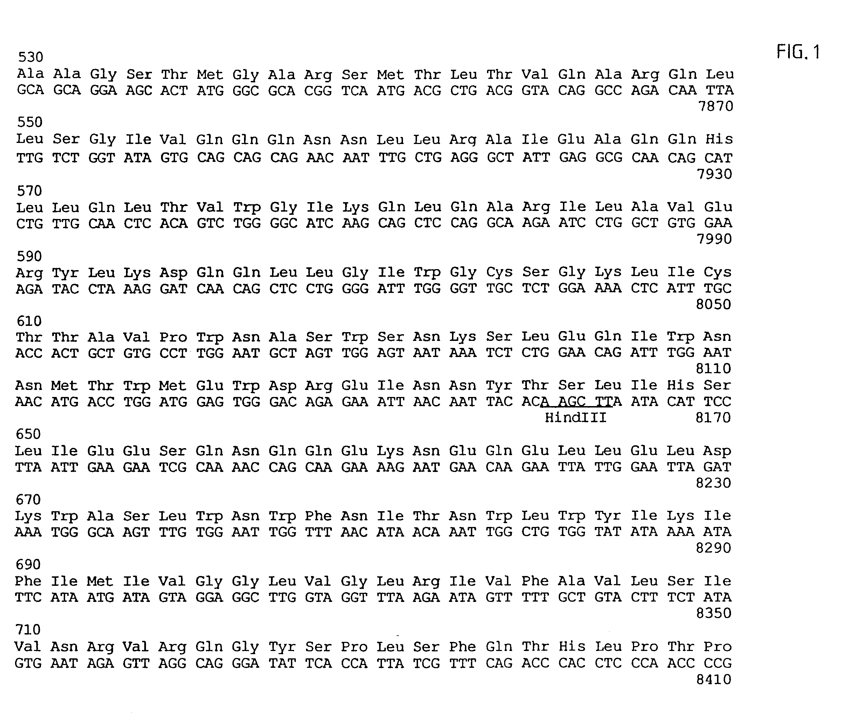

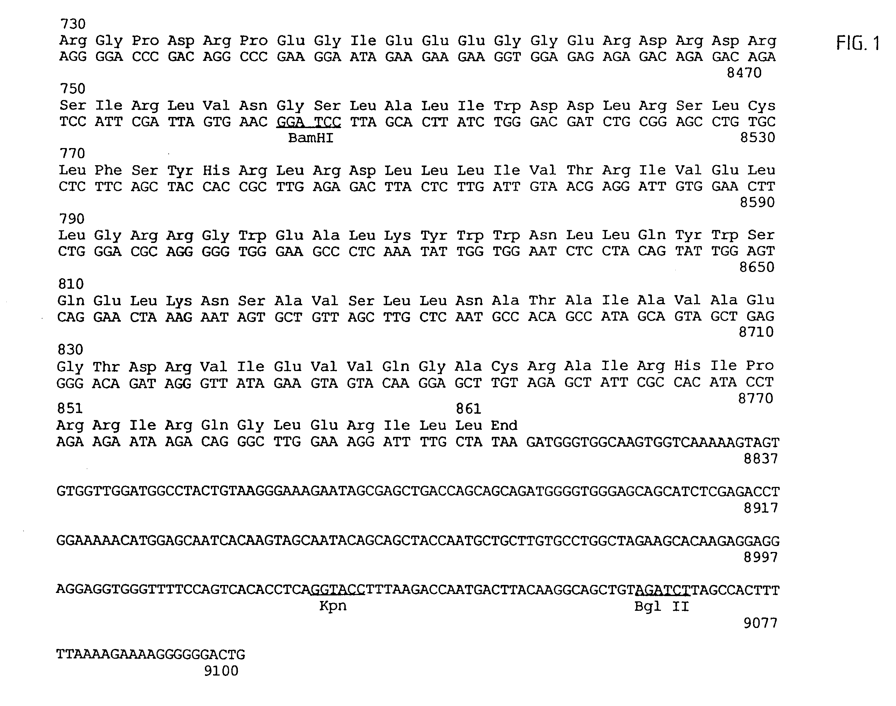

Fig. 1 illustrates the nucleotide sequences of the full-length envelope gene of the LAV-1a isolate. The nucleotide sequence is shown together with the predicted amino acid sequence. Sequence information was obtained from GENBANK. Numbering of nucleotides is from the first nucleotide of the presumed ATG initiation codon. Amino acids are numbered from the ATG initiating codon. Regions corresponding to the signal peptide extracellular glycoprotein (gp120), and transmembrane glycoprotein (gp41) are shown together with proposed peptide cleavage sites and asparagine-linked glycosylation sites. Pertinent restriction enzyme sites are listed below the nucleotide sequences.

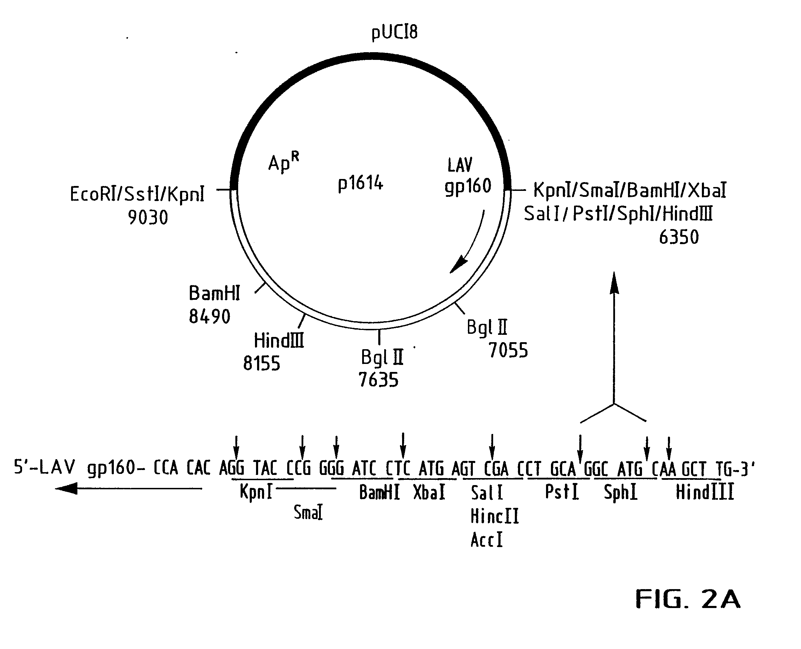

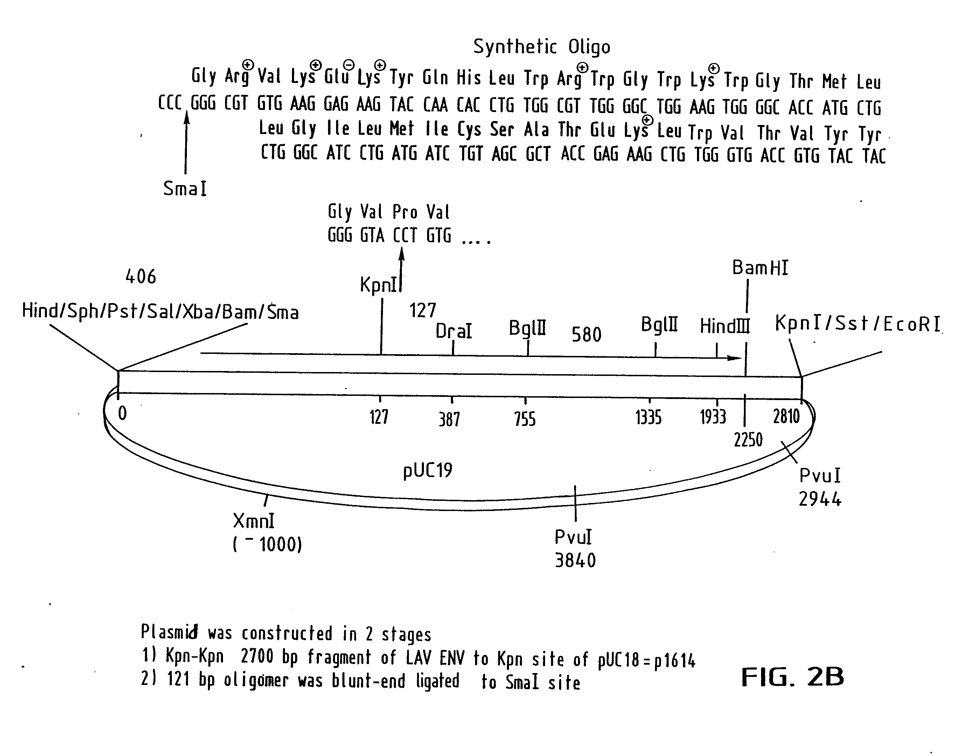

Fig. 2 which includes Fig. 2A and Fig. 2B illustrates structures of recombinant plasmids p1614 and p1774. Plasmid p1774 contains the entire coding sequence of the LAV env gene. The plasmid was constructed in two stages: (a) the 2686 bp KpnI fragment of LAV env gene was isolated from p1614 and cloned into the KpnI site pUC18 such that the SmaI site of pUC18 was upstream from the env gene sequence; (b) the 121 bp synthetic oligomer was blunt-end ligated to the SmaI site. Arrows indicate the polarity of coding sequences. Relevant restriction endonuclease sites are shown. Nucleotide sequence of synthetic oligomers prepared for this invention were constructed on a Pharmacia Gene Assembler and based on the predicted amino acid sequence of the region of LAV using the preferred codon usage of the polyhedrin gene.

Fig. 3 which includes Fig. 3A, Fig. 3B and Fig. 3C illustrates structures of insertion vectors. Insertion vectors MGS-3, MGS-3+2, MGS-4 and MGS-5 are shown with relevant restriction endonuclease sites and nucleotide sequence. The polarity of the promoter sequences is from left to right.

Fig. 4 illustrates predicted secondary structure and hydrophilicity patterns of precursor gp160 as generated from computer analysis using program of Chow and Fastman (1974, Biochemistry 13.222).

Fig. 5 illustrates approaches for the construction of recombination vectors. Plasmids shown here have been described, or have been designated a letter (e.g. A) which corresponds to constructs described in Tables 1 and 2.

The insertion vector MGS-1 was constructed from an EcoRI-I restriction fragment clone of DNA isolated from a plaque purified AcMNPV isolate.

4

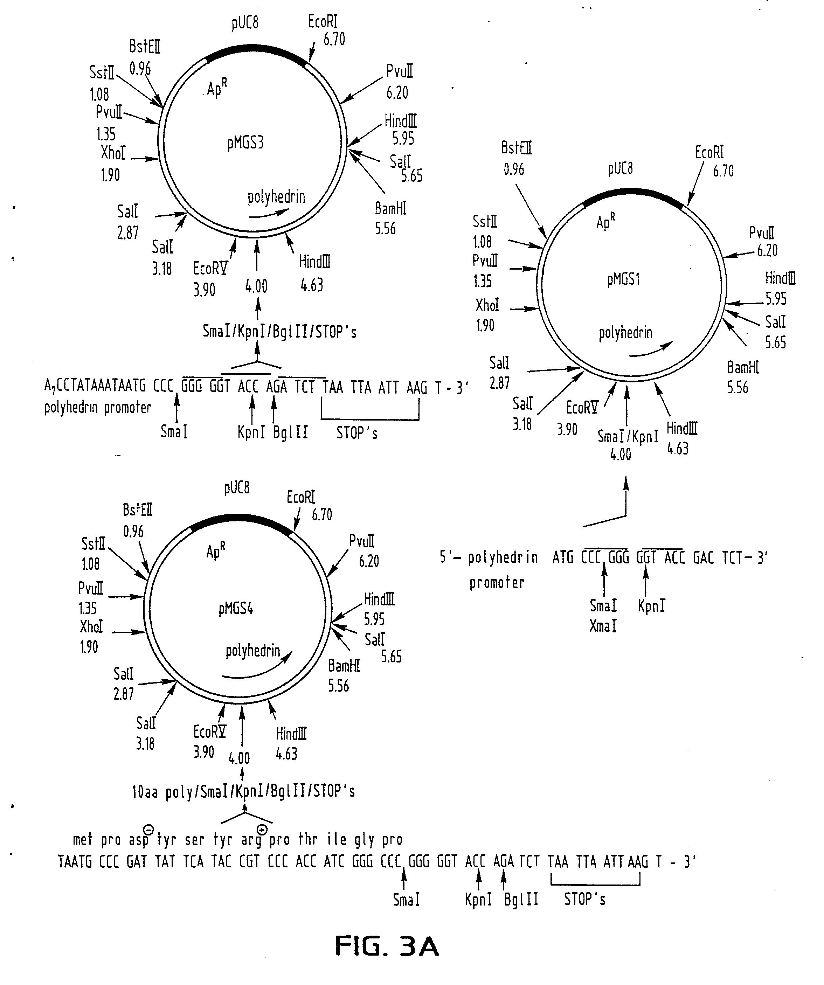

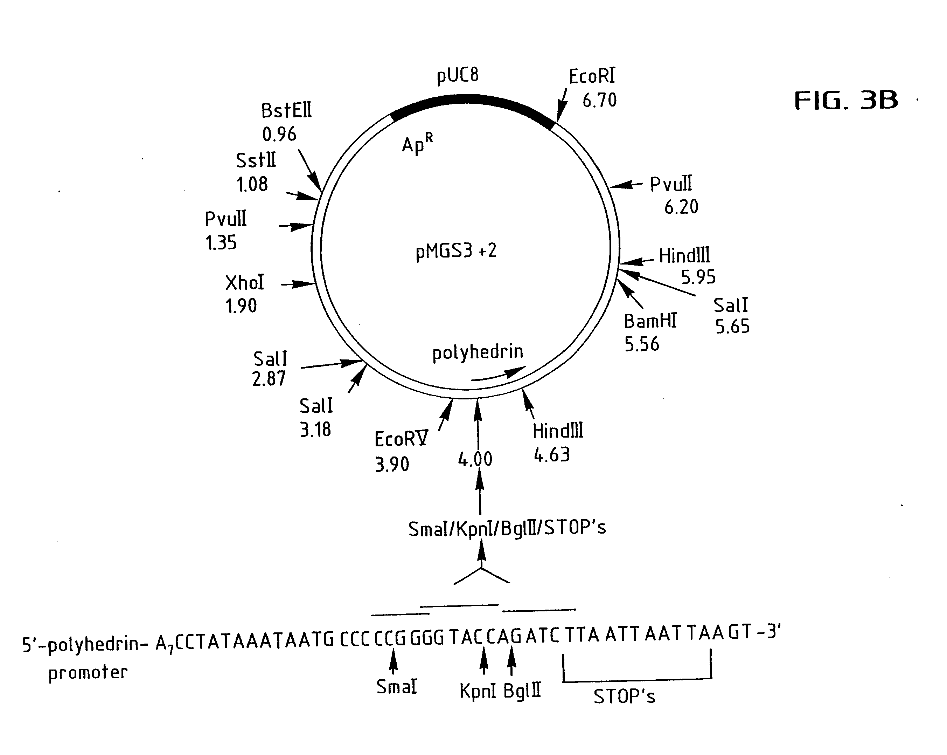

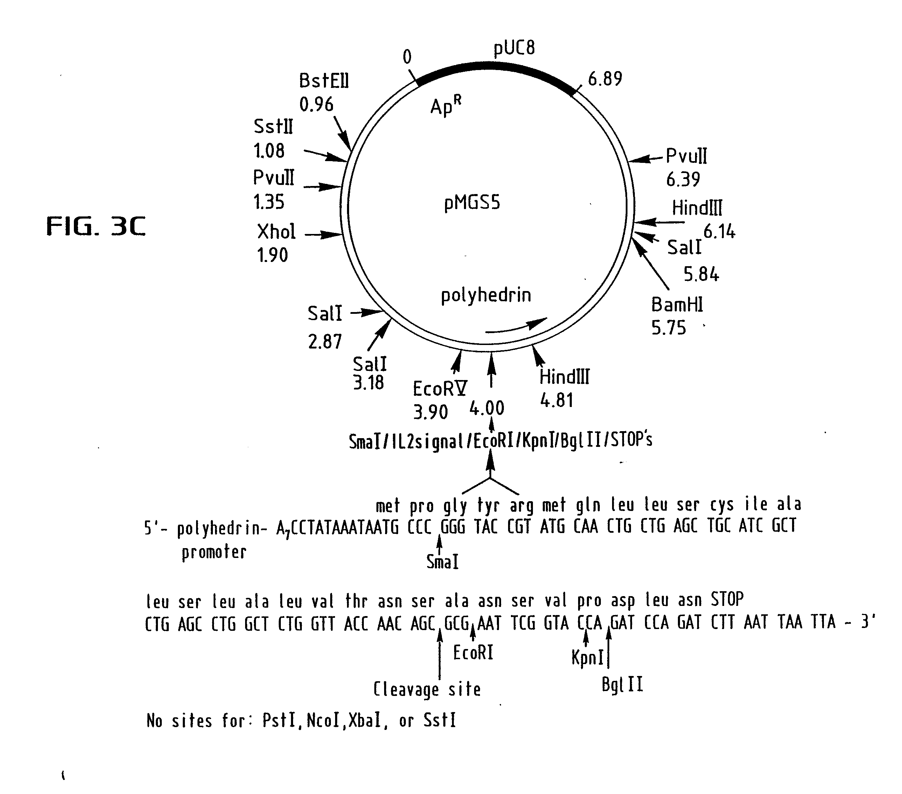

Insertion vector MGS-1 consists of the following structural features (see Fig. 3): 4000 bp of sequence upstream from the ATG initiating codon of the polyhedrin gene; a polylinker introduced by site directed mutagenesis, which consists of an ATG initiating codon, and restriction sites SmaI and KpnI; 1700 bp of sequence extending from the KpnI restriction site (which is internal to the polyhedrin gene) through to the terminal EcoRI restriction site of the EcoRI-I clone.

Insertion vector MGS-3 is identical to MGS-1 except that the polylinker consists of restriction sites SmaI, KpnI, BglII and a universal stop codon segment.

Insertion vector MGS-3+2 is identical to MGS-3 except that it contains two additional cytosine residues at position +3 of MGS-3 and has one less guanosine residue at position +4 of MGS-3. The end result is that the codon-frame is shifted by one nucleotide relative to MGS-3.

Insertion vector MGS-4 contains the same structural design as described for MGS-3 except that it was constructed with a synthetic polylinker that includes sequences coding for the first 10 amino acids of the N-terminal portion of the polyhedrin gene, followed by restriction site for SmaI, KpnI, BGIII and a universal stop codon segment. This vector was constructed based on the observation that increased levels of expression can be obtained if the first 14 amino acid codons of the N-terminal end of the polyhedrin gene are fused to the N-terminal end of the foreign gene (Smith et al. 1983).

Insertion vector MGS-5 contains the same structural design as described for MGS-3 except that it was constructed with a synthetic polylinker that includes sequences coding for the cleavable signal peptide of IL-2 followed by restriction sites for EcoRI, KpnI, BGIII and a universal stop codon segment. This vector was used to supply internal HIV env sequences with a peptide signal sequence which has been shown to be effectively recognized and removed in insect cells (Smith et al. 1985).

Construction of baculovirus recombinants bearing LAV encoding sequences

A recombinant plasmid, designated NA-2, which consisted of a 21.8 Kb segment of an entire AIDS provirus inserted into pUC18, was employed. This clone was reportedly infectious since it could produce virus following transfection of certain human cells. The complete envelope gene sequences contained in NA-2 were derived from the LAV strain of HIV (Barre-Sinoussi et al. 1983).

The envelope gene of LAV contains an open reading frame that codes for 861 amino acids starting with the met codon (Wain-Hobson et al. 1985). The HTLV-III BHIO strain contains 856 codons (Starich et al. 1986).

The signal peptide sequence consists of 30 amino acids (2 of which differ from BHIO); the extra cellular portion consists of 486 amino acids (14 of which differ from BHIO); and the transmembrane region consists of 345 amino acids (5 of which differ from BHIO). Fig. 1 shows the nucleotide and deduced amino acid sequences of the LAV env gene used in these studies. Amino acid residues are numbered beginning with the presumed initiating met condon. Amino acid residue referred to herein correspond with the numbers shown in Fig. 1.

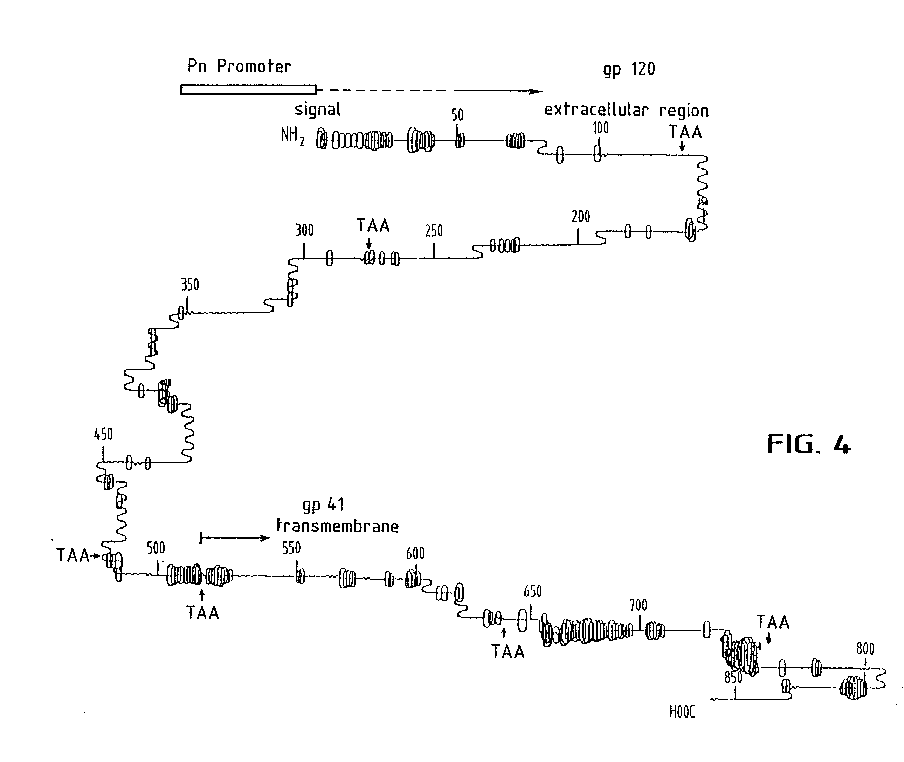

It was decided to express a variety of domains of the HIV-env gene, in addition to the entire gp160 polypeptide. This decision was based on several factors, including protein structure and reported heterogeneity of AIDS retrovirus isolates (Benn et al. 1985, Hahn et al. 1985) A computer program that predicts the secondary structure of proteins superimposed with values for hydrophilicity (Chow and Fastman 1984) was employed. The analysis, shown in Fig. 4, revealed several hydrophilic domains containing Beta turns. Such domains have been shown to be associated with antigenic epitopes and structural positioning (Westhoff et al. 1984). Based on these results and the presence of convenient restriction sites, it was decided to express a variety of C-terminal truncated forms of the envelope protein indicated in Fig. 4. The advantage of such constructions is that progressive deletions beginning with the C-terminal hydrophobic domain were obtained. In addition, it was also decided to express the region spanned by gp41 coding sequences. At least two (2) immunodominant epitopes are included in the regions to be expressed. For these constructs there was designed a vector which included the cleavable signal for IL-2. It had been shown recently that the IL-2 signal peptide resulted in correct cellular processing association of the IL-2 gene as expressed from a recombinant baculovirus genome in infected cells (Smith et al. 1985).

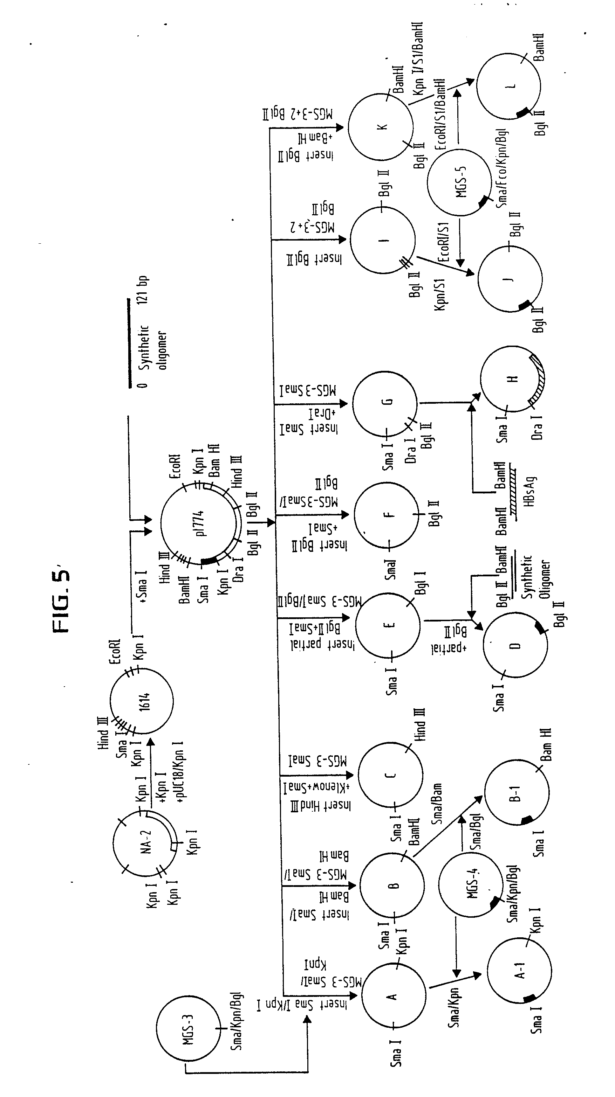

The cloning strategy devised for the expression of HIV-env sequences is shown in Fig. 5.

The envelope gene was initially isolated from NA-2 as a 3846 bp EcoRI/SacI restriction fragment and cloned into the EcoRI/SacI restriction site of pUC19. The resultant plasmid was designated as p708. The envelope gene was subsequently reisolated as a 2800 bp KpnI restriction fragment and cloned into the KpnI restriction site of pUC18. The resulting clone was designated p1614 (see Fig. 2A). This kpnI restriction fragment contained a slightly truncated piece of the envelope gene such that 121 bp of the N-terminal corresponding sequence was missing. This missing part in the gene. which included the signal peptide sequences, was replace by insertion of a double-stranded synthetic oligomer which was designed from the LAV amino acid sequence using preferred polyhedrin gene codon usage. To faciliate further manipulation, a new SmaI restriction sequence was concomitantly introduced in place of the ATG initiating codon. The ATG initiation codon will be supplied by the insertion vector. The resultant plasmid was designated as p1774 and is shown in Fig. 2B.

Restriction fragments from p1774 containing codon sequences of various domains of the AIDS envelope were cloned into the MGS vectors such that the ATG initiating codon of the insertion vector was in-frame with the codons of the envelope gene. The following constructs were made (see Fig. 5).

A. Full-length gp160 cloned as a SmaI/partial KpnI digest fragment inot MGS-3 at its SmaI site. This clone contains all the coding sequences of gp160 and uses its authentic translation termination codon.

A1. Full-length gp160 cloned as a SmaI/partial KpnI digest fragment into MGS-4 at its SmaI/KpnI site. This clone contains all the coding sequences of gp160 and uses its authentic translation termination codon.

B. Truncated gp160 cloned as a SmaI/BamHI restriction fragment in the SmaI/BglII restriction site of MGS-3. This clone contains sequences coding for amino acids 1 through 757 of gp160 and uses a termination codon supplied by the MGS-3 vector.

B1. Truncated gp160 as a SmaI/BamHI restriction fragment in the SmaI/BglII restriction site of MGS-4. This clone contains sequences coding for amino acids 1 through 757 of gp160 and uses a termination codon supplied by the MGS-4 vector.

C. Truncated gp160, cloned as a SmaI/filled in HindIII restriction fragment in the SmaI site of MGS-3. This clone contains sequences coding for amino acids 1 through 645 of gp160 and uses a termination codon supplied by the MGS-3 vector.

D. Full-length gp120, cloned as a SmaI/partial BglII restriction fragment to which a synthetic DNA linker had been added to the BglII site to fill in the sequences from that BglII site (at amino acid 472 codon) to the final C-terminal codon of gp120. This clone contains sequences from amino acid 1 through 516, representing the entire gp120 coding sequence. Translational termination is at a TAA supplied by the MGS-3 vector.

E. Truncated gp120, cloned as a SmaI/partial BglII restriction fragment into the SmaI/BglII restriction site of MGS-3.

F. Truncated gp120, cloned as a SmaI/BglII restriction fragment to the SmaI/BglII site of MGS-3. This clone contains sequences coding from amino acids 1 through 279 and uses a termination codon supplied by the MGS-3 vector.

G. Truncated gp120, cloned as a SmaI/DraI restriction fragment cloned into the SmaI site of MGS-3. This clone contains sequences coding for amino acids 1 through 129 and uses a termination codon supplied by the MGS-3 vector.

H. the above SmaI/DraI construct to which the sequences coding for the HBsAg were introduced as a BamHI fragment to the BglII site located downstream on the vector. This clone contains sequences coding for the first 129 N-terminal amino acids of gp120 followed by in-frame sequences coding for the HBsAg.

I. gp41 cloned as a BglII restriction fragment into the BglII site of MGS-3. This clone contains sequences coding for amino acids 472 through to the C-terminal end of gp160.

J. gp41 cloned as a SmaI/KpnI fragment isolated from P3156 and cloned into MGS-5 vector at the trimmed EroRI site and KpnI site. This clone contains sequences coding for the signal peptide of IL-2 fused to sequences coding for amino acids 473 through to the C-terminal end of gp160.

K. Truncated gp41 cloned as a BglII/BamHI fragment into the BglII site of MGS-3. This clone contains sequences coding for amino acids 472 through to 757 of gp160 and uses a termination codon supplied by the MGS-3 vector.

L. Truncated gp41 cloned as a KpnI/BamHi fragment isolated from p3166 and cloned into the KpnI/BamHI site of pMGS-5. This clone contains sequences coding for the signal peptide of IL-2 fused to sequences coding for amino acids 472 through to 757 of gp160.

## Preparation and Selection of Recombinant Baculovirus

The HIV env-gene recombination plasmids were calcium phosphate precipitated with AcMNPV and added to uninfected Spodoptera frugiperda cells. The chimeric genes were then inserted into the AcMNPV genome by homologous recombination. Recombinant viruses were identified by an occ⁻ plaque morphology. Such plaques exhibit an identifiable cytopathic effect but no nuclear occlusions. Two additional successive plaque purifications were carried out to obtain recombinant virus. Recombinant viral DNA was analyzed for site-specific insertion of the HIV env sequences by comparing their restriction and hybridization characteristics to wild-type viral DNA.

## Expression of HIV env from recombinant baculoviruses in infected insect cells

Expression of HIV-env sequences from the recombinant viruses in insect cells should result in the synthesis of primary translational product in the form of a pre-pro-protein containing all the amino acids coded for from the ATG initiating codon of the expression vector downstream from the polyhedrin promoter. This primary product will consist of amino acids translated from the codons supplied by the recombination vector. For example, the primary translation product of construct A should read Met-Pro-Gly-Arg-Val at the N-terminus. The Met-Pro-Gly condons are supplied as a result of the cloning strategy.

Two potential processing sites for the cleavage of the env precursor glycoprotein would firstly remove 30 amino acid residues of the signal peptide at the N-terminal end and secondly generate a large transmembrane protein containing 345 amino acids, and an extracellular portion of 486 amino acids. It is generally believed that recognition and cleavage of the signal peptide is required for efficient cell processing.

To determine expression of HIV env gene sequence from recombinant baculoviruses, there were infected cultures of insect cells with each of the various recombinant viruses in the presence of ³⁵S-methionine, ³⁵S-cystine or ³H-mannose at late times in infection. Labeled cell extracts were displayed by SDS polyacrylamide gel electrophoresis (PAGE) and autoradiography.

Three types of serum were used to evaluate the authenticity of the recombinant protein produced in insect cells infected with HIV recombinant baculoviruses:

1. HIV-positive human sera supplied by the Center for Disease Control (CDC, Atlanta, Georgia) as an HIV-positive reference standard.

2. HIV-negative human sera, also supplied by the CDC as an HIV-negative reference standard.

3. polyclonal antibody raised in goats to gel purified gp120 envelope protein prepared from purified infectious HTLV-III virus.

The results are summarized in Tables 1 and 2:

Table 1.

| Isolate No. | | Description | Serum | | |
|---|---|---|---|---|---|
| | | | HIV-neg | HIV-pos | gp120-pos |
| Controls | | | | | |
| uninf. Sf cells | | | − | − | − |
| wt inf Sf cells | | | − | − | − |
| Recombinants | | | | | |
| A. | 2863 | whole gp160 1-861 | − | + | + |
| A1. | 3715 | | nd | nd | nd |
| B. | 3046 | truncated gp160 1-757 | − | + | + |
| B1. | 3540 | | nd | nd | nd |
| C. | 3774 | truncated gp160 1-645 | nd | nd | nd |
| D. | 4646 | gp120, 1-516 | nd | nd | nd |
| E. | 2040 | truncated gp120 1-472 | − | + | + |
| F. | 2165 | truncated gp120 1-279 | − | − | +/− |
| G. | 2196 | truncated gp120 1-129 | − | − | − |
| H. | 3076 | amino acid 1-129 fused to HBsAg | nd | nd | nd |
| I. | 3156 | gp41, 472-861 | − | + | + |
| J. | 4585 | IL-2 signal/gp41 | nd | nd | nd |
| K. | 3166 | truncated gp41 472-757 | − | + | + |
| L. | | IL-2 signal/ truncated gp41 | − | − | − |

Table 2

Summary of Isolate, Predicted and Observed Results

| Construction/ Isolate No. | | Envelope amino acids[1] | Predicted Size[2] | | Observed Size[3] |
|---|---|---|---|---|---|
| | | | Unglyocylated | Glyocylated | |
| A. | 2863 | whole gp160 1-861 | 93,200 56,000 37,000 | 160,000 120,000 41,000 | 160,000 120,000 41,000 |
| A1. | 3715 | | as in A | as in A | as in A |
| B. | 3046 | truncated gp160 1-757 | 82,000 56,000 26,000 | 150,000 120,000 30,000 | 150,000 120,000 30,000 |
| B1. | 3540 | | as in B | as in B | as in B |
| C. | 3774 | truncated gp160 1-645 | 69,000 56,000 14,000 | 130,000 120,000 18,000 | |
| D. | 4646 | gp120, 1-516 | 56,000 | 120,000 | N.D. |
| E. | 2040 | truncated gp120 1-472 | 51,000 | 110,000 | 110,000 90,000 |
| F. | 2165 | truncated gp120 1-279 | 30,000 | 60,000 | 60,000 |
| G. | 2196 | truncated gp120 1-129 | 12,000 | 15,000 | 15,000 |
| H. | 3076 | amino acid 1-129 fused to HBsAg | 35,000 | 40,000 | |
| I. | 3156 | gp41, 472-861 | 42,000 | 46,000 | |
| J. | 4585 | IL-2 signal/gp41 | 42,000 | 46,000 | N.D. |
| K. | 3166 | truncated gp41 472-757 | 30,000 | 33,000 | N.D. |
| L. | | IL-2 signal/ truncated gp41 | 30,000 | 33,000 | N.D. |

1. Amino acid residues predicted to be present in unprocessed pre-pro gene product as deterined from nucletide sequence.

2. Estimated from amino acid residues present and predicted processed forms.

3. Major immunoreactive polypeptides.

Recombinant gp160 proteins have been successfully used in typical diagnostic assays such as ELISA and radioimmunoprecipitation in addition to Western Blot analyses.

## PURIFICATION HIV ENVELOPE PROTEINS

Recombinant HIV envelope proteins are produced in S. frugiperda cells during the 4-5 days after infection with an HIV recombinant AcNPV virus. The majority of the expressed protein is associated with the infected cells. Because all of the envelope HIV gene products described herein have similar properties, that is, they are primarily cell-associated and are glycoproteins, one purification method can be used for all of the HIV envelope gene products described in this application or other similar constructions. The following is an example of how the recombinant gp160 protein produced from the expression vector Ac3046 is purified.

S. frugiperda cells are infected with the recombinant Ac3046. At 4-5 days after infection, the cells are collected and washed free of the cell culture medium. The cells are first fractionated into nuclear and cytoplasmic membrane fractions using standard procedures. The glycoprotein fraction contains the gp160 protein, is solubilized and the glycoproteins purified using lentil lectin affinity chromatography using standard procedures. The glycoprotein fraction contains the gp160 protein and at this stage the gp160 protein is 25%-50% of the total glycoprotein fraction as judged by SDS-polyacrylamide gel analysis. To further purify the gp160, the glycoprotein fraction is run through a molelcular sieve liquid chromatography column. The gp160 protein elutes from the column as a high molecular weight fraction and the gp160 protein is approximately 90% of the total protein in the high molecular weight fraction.

Of some interest in connection with this invention is the article entitled "AIDS Virus Env Protein Expressed from the Recombinant Vaccinia Virus" by M. P. Kieny et al, published in Bio/Technology, Vol. 4, September 1986, pp. 790-795. This paper discloses that the env coding sequence for the env protein of LAV was introduced into vaccinia virus vector. The resulting live recombinant virus VVTGeLAV then determines the production of env protein in infected mammalian cells. This recombinant protein reacts with sera from AIDS patients and appears to be processed and glycosylated in a manner identical to authentic envof LAV retrovirus. Also, the inoculation of mice with VVTGeLAV elicits high titre values of antisera recognizing vaccinia determinants but only low titres of antibody recognizing env proteins of LAV. Cells infected with the recombinant virus rapidly liberate the processed form of env protein into the culture medium. This approach, however, for the production and utilization of env protein itself, such as in a vaccine to elicit an immune response against the LAV or HIV virus, is not completely satisfactory, particularly because of the use of the vaccinia virus as the vector.

Also of interest in connection with this invention is the article entitled "Production of Human Beta Interferon in Insect Cells Infected with Baculovirus Expression Vector" by G. E. Smith et al, Molecular and Cellular Biology, Vol. 3, No. 12, pp. 183-192, December 1983, and the article entitled"Strong and Regulated Expression of Escherichia coli Beta-Galactosidase in Insect Cells with a Baculovirus Vector" by G. D. Pennock et al, Molecular and Cellular Biology, Vol. 4, No. 3, pp. 399-406, March 1984. Also of interest is copending, coassigned patent application Serial No. 810,938 filed December 18, 1985. European Patent Publication No. 0 127 839 published December 12, 1984 and European Patent Publication No. 0 155 474 published September 25, 1985 are also of interest in connection with this invention. The disclosures of these articles and these patent publications and the patent application are herein incorporated and made part of this disclosure.

Since Human Acquired Immune Deficiency Syndrome (AIDS) is epidemic in the United States, Central Africa, Europe and in other areas of the world, this invention is of great importance. As indicated hereinabove, the causative agent of this disease, AIDS, is a retrovirus termed Human Imunodeficiency Virus (HIV) and which has also been variously termed Lymphadenopathy Virus (LAV), Human T-Cell Leukemia Virus Type III (HTLV-III) or AIDS-Related Virus (ARV). The structure and gene organization of several AIDS viruses have been elucidated from the complete nucleotide sequence of molecular clones and direct sequencing of viral proteins. The HIV envelope gene (env) codes for a 160,000 molecular weight glycoprotein and is referred to as gp160. In virus infected cells the gp160 precursor is cleaved at a conserved sequence of basic amino acids to produce an N-terminal glycoprotein gp120 and a smaller C-terminal protein gp41. HIV glycoproteins, gp160, gp120 and gp41, contain approximately 833, 488 and 345 amino acids, respectively.

The mature gp120 is associated with the virus envelope and is thought to be external, whereas gp41 has two long stretches of hydrophobic residues and one or both of these may traverse the viral envelope. The gp 160 precursor and mature gp120 and gp41 proteins are detected by antisera from most exposed individuals. Also, it has been demonstrated that the gp120 protein binds to the T4 molecule on the cell surface of T helper/inducer lymphyocytes and that the HIV gp160 protein is able to induce syncytium formation with cells that express the T4 receptor protein. It is also known that the 104 carboxyterminal amino acids on gp160 are not required for fusion of T4 + · T-lymphocytes.

The AIDS virus envelope glycoprotein of HIV gp160 in accordance with this invention is expressed from an AIDS virus env gene that was cloned from an infectious HIV isolate. The HIV env gene used for expression codes only for the sequences found in the native HIV env gene. The HIV env gene was inserted into the recombinant baculo-virus such that expression would result in a mature protein that contained no altered or additional amino acids. Of the several recombinants made, one contains the complete HIV env gene and another is complete except for a small deletion of the sequence for approximately 100 amino acids at the C-terminus of gp160. This deletion was made to help stabilize the expressed recombinant gp160 protein and does not remove the two hydrophobic domains present in gp41.

The HIV gp160 was produced, as described hereinabove, in an insect cell expression system and, also as suggested hereinabove, is free of contaminating mammalian cell proteins. Expression and purification of the gp160 protein was carried out under conditions to maintain the native protein structure and its biological activity. The resulting expressed HIV env protein was demonstrated by immunoprecipitation, Western blot and ELISA assays to be highly reactive with AIDS patients' sera.

The HIV gp160 protein of this invention has been produced in various amounts, purity and form, such as in a sterile aqueous buffer at a concentration of about 100 micrograms envelope protein per milliliter at a purity of greater than 50% as determined by SDS-polyacrylamide FPLC analysis. The HIV gp160 protein has been found to be stable for at least six weeks at 4°C. This protein has been provided in amounts or volumes for single use and is satisfactorily stored at -70°C. Desirably, repeated cycles of freezing and thawing should be avoided and dilutions should be made with solutions containing suitable proteins or detergent to prevent loss of protein and biological activity. Suitable diluents include 0.1% bovine serum albumin (BSA) or equivalent, 0.1% serum in sterile water or culture medium and 0.1% sodium dodecylsulfate or other suitable detergent in water or buffer. The protein produced in accordance with this invention, HIV gp 160, is usefully employed, as indicated hereabove, and has been packaged in various sizes depending upon the need or intended use, such as packages having 25 micrograms, 50 micrograms or 100 micrograms of protein. The protein is useful for in vitro and other investigations relating to various aspects of AIDS research including vaccine development, Western blotting, ELISA, receptor binding, immunoprecipitation, antisera production, syncytium formation and other uses including physical studies and as a reference standard.

Western blot analysis is one of the most specific and sensitive methods available for the detection of AIDS antibodies and is often employed in various aspects of AIDS research and, as mentioned hereinabove, the gp160 precursor and the mature gp120 and gp41 proteins are detected by antisera from AIDS seropositive individuals. Accordingly, in one embodiment of this invention the AIDS virus envelope or env protein is applied onto a suitable substrate, ceramic, glass, polystyrene plastic plates, wells or film, such as nitrocellulose membrane strips, for use in connection with the detection of AIDS antibodies.

Specifically, in accordance with this embodiment of the invention, electrophoretically separated AIDS recombinant envelope protein, HIV gp160, is impregnated onto nitrocellulose membrane strips for use as Western Blot or blotting strips. Since the HIV env gp is produced in accordance with this invention in an insect cell expression system, it is free of contaminating mammalian cell proteins. This material deposited on nitrocellulose strips for use as Western blotting strips has been found to be highly reactive with AIDS patients sera and the HIV gp160 deposited on nitrocellulose strips has been tested successfully with human AIDS positive sera at dilutions of 1/100 to 1/10,000. The specifically bound antibody thereon can be detected with either enzyme-linked reagents, such as alkaline phosphatase or peroxidase, conjugated second antibodies or the specific anti-antibodies or radioiodinated protein A. The immunological procedures for Western blot analysis employing these special embodiments of this invention, i.e. the carriers or HIV env protein impregnated blot strips for Western blot analysis, are well known.

The HIV gp160 strips or carriers have been prepared in accordance with this invention in disposable trays with a suitable number, such as eight strips in eight separate wells. All the incubations can be done in the tray and a lid is supplied so that the trays can be used as a convenient storage container for the developed Western blots or detection results. Each such strip provided in accordance with this invention is impregnated with recombinant HIV gp160 envelope protein which was resolved by electrophoresis on a 10% SDS polyacryalmide gel and then transferred electrophoretically to nitrocellulose membranes or strips or carriers. The resulting product is useful for detecting AIDS envelope antibodies in vitro for animal and cell or tissue culture investigations relating to various aspects of AIDS research including Western blotting for detecting AIDS antibodies, and in quality control, blood bank screening and diagnosis of AIDS. The nitrocellulose membrane carriers or strips have been found to be stable for at least 6 weeks at room temperature and are suitably stored, such as in the trays, in a cool dry place.

The HIV gp160 ELISA plates have been prepared in accordance with this invention in· disposable trays with a suitable number, such as 96 separate wells. All of the incubations are done in the trays. Applied to each well is a suitable quantity of HIV gp160, such as 100 ul purified HIV gp 160 at a concentration of 1 ug HIV gp160 per milliliter. The HIV gp160 protein is allowed to attach to the plastic in the wells for a suitable time, such as overnight at 4°C. The remaining solution was then removed from each well in the ELISA plates and the ELISA plates were allowed to dry at room temperature. The resulting plates with the HIV gp160 applied to the wells have been found to be stable for at least 6 weeks at room temperature and are suitably stored, such as in the trays, in a cool dry place. The resulting product is useful for the detection of AIDS envelope antibodies or antigen for animal and cell or tissue culture investigations relating to various aspects of AIDS research including ELISA assays, and screening and diagnostics of human sera for AIDS antibody or antigen.

The following publication references are cited in support of this invention. The disclosures of these references are herein incorporated and made part of this disclosure.

Barin, F., McLane, M.F., Allan, J. S. and Lee, T.H. 1985. Virus envelope proteins of HTLV-III represents the major target antigen for antibodies in AIDS patients. Science 228:1094-1096.

Barre-Sinoussi, F., Chermann, J.C. Rey, F., Nugeybe, M.T., Chamaret, S., Gruest, J., Dauguet, C., Axler-Blin, C., VezinetBrun, F., Rouzioux, C., Rozenbaum, W., and Montagnier, L. 1983. Isolation of a T-lymphotropic retrovirus from a patient at risk of acquired immune deficiency syndrome (AIDS). Science 220:868-870.

Chakrabarti, S., Robert-Guroff, M., Wong-Staal, F., Gallo, R.C., Moss, B. 1986. Expression of HTLV-III envelope gene by a recombinant vaccinia virus. Nature 320:535-537.

Francis, D.P., Petricciani, J.C. 1985. The prospects for and pathways toward a vaccine for AIDS. New Eng. Journal of Med. 1586-1590.

Hu, Shiu-Lok, Kosowski, S.G., Dairymple, J.M. 1986. Expression of AIDS virus envelope gene recombinant vaccinia viruses. Nature 320:537-540.

Iddekinge, B.J.L. Hooft van, G.E. Smith, and M.D. Summers. 1983. Nucleotide sequence of the polyhedrin gene of Autographa californica nuclear polyhedrosis virus. Virology 131:561.

Kennedy, R.C., Henkel, R.D., Pauletti, D., Allan, J.S., Lee, T.H., Essex, M., Dreesman, G.R. 1986. Antiserum to a synthetic peptide recognizes the HTLV-III envelope glycoprotein. Science 231:1556-1559.

Kieny, M.P., Rautmann, G., Schmitt, D., Dott, K., Wain-Hobson, S., Alizon, M., Girard, M., Chamaret, S., Laurent, A., Montagnier, L., Lecocq, J-P. 1986. AIDS virus env protein expressed from a recombinant vaccinia virus. Research Papers 4:790-795.

Kozak, M. 1986. Point mutations define a sequence flanking the AUG initiator codon that modulates translation by eukaryotic ribosomes. Cell 44:283-292.

Lasky, L. A., Groopman, J.E., Fennie, C.W., Benz, P.M., Capon, D.H., Dowbenko, D.J., Nakamura, G.R., Nunes, W.M., Renz, M.E., Berman, P.W. 1986. Neutralization of the AIDS retrovirus by anitbodies to a recombinant envelope glycoprotein Science 233:209-212.

Levy, J.A., Hoffman, A.D., Kramer, S.M., Shimabururo, J.M., and Oskiro, L.S. 1984. Isolation of lymphocytopathic retroviruses from San Francisco patients with AIDS. Science 225:840-842.

McDougal, J.S., Kennedy, M.S. Sligh, J.M., Cort, S.P., Mawle, A., and Nicholson, J. K. A., 1986. Binding of HTLV-III/LAV to T4⁺ T cells by a complex of the 110K viral protein and the T4 molecule. Science 231:382-388.

Montagnier, I., Clavel, F., Krust, B. 1985. Identification and antigenicity of the major envelope glycoprotein of lymphadenopathy-associated virus. Virology 144:283-289.

Muesing, M.A., Smith, D.H., Cabradella, C.D. et al. 1985. Nucleic acid structure and expression of the human AIDS/lymphadenopathy retrovirus. Nature 313:450-458.

Pennock, G.D., C. Shoemaker, and L,K. Miller. 1984. Strong and regulated expression of Escherichia coli B-galactosidase in insect cells with a baculovirus vector. Mol. Cell. Biol. 4:399.

Popovic, M., Sarngadharan, M.G., Read, E., and Gallo, R.C. 1984. Detection, isolation, and continuous production of cytopathic retroviruses (HTLV-III) from patients with AIDS and pre-AIDS. Science 224:497-500.

Ratner, L., Hhaseltine, W., Patarca, R., et al. 1985. Complete nucletide sequence of the AIDS virus, HTLV-III. Nature 313:277-284.

Robey, W.G., Safai, B., Oroszlan, S., et al. 1985. Characterization of envelope and core structural gene products of HTLV-III with sera from AIDS patients. Science 228:595-595.

Smith, G.E., M.J. Fraser, and M.D. Summres. 1983a. Molecular engineering of the Autographa Californica nuclear polyhedrosis virus genome: Deletion mutations within the polyhedrin gene.J. Virol. 46:584.

Smith, G.E., M.D. Summers, and M.J. Fraser. 1983b. Production of human beta interferon in insect cells infected with baculovirus expression vector. Mol. Cell. Biol. 3:2156.

Smith, G.E., G. Ju, B.L. Ericson, J. Moschera, H.W. Lahm, and M.D. Summers. 1985. Modification and secretion of human interleukin-2 produced in insect cells by baculovirus expression vector. Proc. Natl. Acad. Science. U.S.A. (in press).

Starich, B.R., Hahn, B.H., Shaw, G.M., McNeely, P.D., Modrow, S., Wolf, H., Parks, E.S., Parks, W.P., Josephs, S.F., Gallo, R.C., Wong-Staal, F. 1986. Identification and Characterization of conserved and variable regions in the envelope gene of HTLV-III/LAV, the retrovirus of AIDS. Cell. 45:637-648.

Summers, and G. Ju. 1985. Production of human c-myc protein in insect cells infected with a baculovirus expression vector. Mol. Cell. Biol. (in press).

Towbin, H., Staehelin, T. and Gordon, T. 1979. Electrophoretic transfer of proteins from polyacrylamide gels to nitrocellulose sheets: procedure nad some applications. Pro. Natl. Acad. Sci. 76:4350-4353.

Wain-Hobson, S., Sonigo, P., Danos, O., Cole, S., and Alizon, M. Nucleotide Sequence of the AIDS virus, LAV. 1985. Cell 40:9-17.

**Claims**

1. AIDS virus env protein expressed from a recombinant insect virus.

2. AIDS virus env gp160 protein, AIDS virus env gp120 protein, AIDS virus env gp41 protein, or an immunogenic fragment of AIDS virus env protein in accordance with Claim 1.

3. Recombinant insect baculovirus having incorporated therein a gene for expression of the AIDS virus env protein, a gene for an immunogenic fragment of AIDS Virus env protein, the gene for AIDS virus env gp160 protein, the gene for AIDS virus env gp120 protein or the gene for AIDS virus env gp41 protein in accordance with Claim 1 or 2.

4. AIDS virus env protein in accordance with Claim 1 incorporated in or fixed onto a substrate or carrier, e.g. a film, a transparent substrate or carrier, or a nitrocellulose film or membrane.

5. An immunogenic fragment of AIDS virus env protein in accordance with Claim 2 incorporated in or fixed onto a solid substrate or carrier.

6. A vaccine against AIDS virus comprising an immunogenic fragment of AIDS virus env protein, AIDS virus env gp160 protein, AIDS virus env gp120 protein, or AIDS virus env gp41 protein in accordance with Claim 2.

7. A diagnostic composition useful for the detection of AIDS antibody comprising an immunogenic fragment of AIDS virus env protein in accordance with Claim 2 and a liquid carrier therefor.

8. A method of detecting AIDS virus antibody which comprises contacting said AIDS virus antibody with AIDS env protein expressed from a recombinant insect virus in accordance with Claim 1 and determining any fixing or reaction between said AIDS virus antibody and said protein.

9. AIDS virus gag protein expressed from a recombinant insect virus.

10. AIDS virus pol protein expressed from a recombinant insect virus.

11. A method of producing AIDS virus env protein which comprises inserting the AIDS virus env protein gene or the AIDS virus envelope gene (env) into the DNA of an insect virus, infecting insect cells or insects with the resulting recombinant virus and culturing the resulting infected insect or insect cells to express or produce the AIDS virus env protein.

12. A method of constructing a recombinant baculovirus expression vector capable of expressing a selected AIDS virus gene or portion thereof in a host insect cell, comprising:

(a) preparing a recombinant insertion vector by inserting a portion of a baculovirus genome into a cloning vehicle and thereafter inserting a selected AIDS virus gene or portion thereof into the modified insertion vector such that the selected AIDS virus gene or portion thereof is expressed under the control of a baculovirus promoter,

(b) transferring the above modified AIDS virus gene or portion thereof into a baculovirus expression vector by mixing the above modified insertion vector with baculovirus DNA, transfecting suitable insect cells, and isolating recombinant viruses that contain the selected AIDS gene or portion thereof.

13. A method for synthesizing a selected AIDS virus polypeptide which comprises infecting a susceptible host insect cell with a recombinant baculovirus vector wherein the baculovirus vector contains one or more AIDS virus protein genes or portions thereof and culturing the infected cell to express the AIDS virus polypeptide.

14. A method for the purification of AIDS env gene polypeptides comprising subjecting said polypeptides to lentil lectin affinity chromatography to isolate glycoproteins, followed by size tractionation of the glycoproteins using molecular sieve chromatography.

15. A vaccine against AIDS disease comprising the AIDS virus env protein of Claim 1 and a physiological carrier therefor.

# FIG. 1

```
                                          1
                                          Met Arg Val Lys Glu Lys Tyr Gln His
AGGTTAATTGATAGACTAATAGAAAGAGCAGAAGACAGTGGCA ATG AGA GTG AAG GAG AAA TAT CAG CAC
                                                                          6250
10                                        <-------------- Signal Peptide ---------
Leu Trp Arg Trp Gly Trp Lys Trp Gly Thr Met Leu Leu Gly Ile Leu Met Ile Cys Ser
TTG TGG AGA TGG GGG TGG AAA TGG GGC ACC ATG CTC CTT GGG ATA TTG ATG ATC TGT AGT
                                                                          6310
--\ /----------- Extracellular Region (gp120) --------->42
Ala Thr Glu Lys Leu Trp Val Thr Val Tyr Tyr Gly Val Pro Val Trp Lys Glu Ala Thr
GCT ACA GAA AAA TTG TGG GTC ACA GTC TAT TAT GGG GTA CCT GTG TGG AAG GAA GCA ACC
                                                KpnI                      6370
50
Thr Thr Leu Phe Cys Ala Ser Asp Ala Lys Ala Tyr Asp Thr Glu Val His Asn Val Trp
ACC ACT CTA TTT TGT GCA TCA GAT GCT AAA GCA TAT GAT ACA GAG GTA CAT AAT GTT TGG
                                                                          6430
70
Ala Thr His Ala Cys Val Pro Thr Asp Pro Asn Pro Gln Glu Val Val Leu Val Asn Val
GCC ACA CAT GCC TGT GTA CCC ACA GAC CCC AAC CCA CAA GAA GTA GTA TTG GTA AAT GTG
                                                                          6490
90
Thr Glu Asn Phe Asn Met Trp Lys Asn Asp Met Val Glu Gln Met Asp Glu Asp Ile Ile
ACA GAA AAT TTT AAC ATG TGG AAA AAT GAC ATG GTA GAA CAG ATG GAT GAG GAT ATA ATC
                                                                          6550
110
Ser Leu Trp Asp Gln Ser Leu Lys Pro Cys Val Lys Leu Thr Pro Leu Cys Val Ser Leu
AGT TTA TGG GAT CAA AGC CTA AAG CCA TGT GTA AAA TTA ACC CCA CTC TGT GTT AGT TTA
                                                                          DraI
130
Lys Cys Thr Asp Leu Gly Asn Ala Ser Asn Thr Asn Ser Thr Asn Thr Asn Ser Ser Ser
AAG TGC ACT GAT TTG GGG AAT GCT AGT AAT ACC AAT AGT ACT AAT ACC AAT AGT AGT AGC
                                                                          6670
150
Gly Glu Met Met Met Glu Lys Gly Glu Ile Lys Asn Cys Ser Phe Asn Ile Ser Thr Ser
GGG GAA ATG ATG ATG GAG AAA GGA GAG ATA AAA AAC TGC TCT TTC AAT ATC AGC ACA AGC
                                                                          6730
```

0 265 785

FIG. 1

```
170
Ile Arg Gly Lys Val Gln Lys Glu Tyr Ala Phe Phe Tyr Lys Leu Asp Ile Ile Pro Ile
ATA AGA GGT AAG GTG CAG AAA GAA TAT GCA TTT TTT TAT AAA CTT GAT ATA ATA CCA ATA
                                                                            6790
190
Asp Asn Asp Thr Thr Ser Tyr Thr Leu Thr Ser Cys Asn Thr Ser Val Ile Thr Gln Ala
GAT AAT GAT ACT ACC AGC TAT ACG TTG ACA AGT TGT AAC ACC TCA GTC ATT ACA CAG GCC
                                                                            6850
210
Cys Pro Lys Val Ser Phe Glu Pro Ile Pro Ile His Tyr Cys Ala Pro Ala Gly Phe Ala
TGT CCA AAG GTA TCC TTT GAG CCA ATT CCC ATA CAT TAT TGT GCC CCG GCT GGT TTT GCG
                                                                            6910
230
Ile Leu Lys Cys Asn Asn Lys Thr Phe Asn Gly Thr Gly Pro Cys Thr Asn Val Ser Thr
ATT CTA AAA TGT AAT AAT AAG ACG TTC AAT GGA ACA GGA CCA TGT ACA AAT GTC AGC ACA
                                                                            6970
250.
Val Gln Cys Thr His Gly Ile Arg Pro Val Val Ser Thr Gln Leu Leu Leu Asn Gly Ser
GTA CAA TGT ACA CAT GGA ATT AGG CCA GTA GTA TCA ACT CAA CTG CTG TTG AAT GGC AGT
                                                                            7030
270
Leu Ala Glu Glu Glu Val Val Ile Arg Ser Ala Asn Phe Thr Asp Asn Ala Lys Thr Ile
CTA GCA GAA GAA GAG GTA GTA ATT AGA TCT GCC AAT TTC ACA GAC AAT GCT AAA ACC ATA
                             BglII                                           7090
290
Ile Val Gln Leu Asn Gln Ser Val Glu Ile Asn Cys Thr Arg Pro Asn Asn Asn Thr Arg
ATA GTA CAG CTG AAC CAA TCT GTA GAA ATT AAT TGT ACA AGA CCC AAC AAC AAT ACA AGA
                                                                            7150
310
Lys Ser Ile Arg Ile Gln Arg Gly Pro Gly Arg Ala Phe Val Thr Ile Gly Lys Ile Gly
AAA AGT ATC CGT ATC CAG AGG GGA CCA GGG AGA GCA TTT GTT ACA ATA GGA AAA ATA GGA
                                                                            7210
330
Asn Met Arg Gln Ala His Cys Asn Ile Ser Arg Ala Lys Trp Asn Ala Thr Leu Lys Gln
AAT ATG AGA CAA GCA CAT TGT AAC ATT AGT AGA GCA AAA TGG AAT GCC ACT TTA AAA CAG
                                                                            7270
```

0 265 785

350
Ile Ala Ser Lys Leu Arg Glu Gln Phe Gly Asn Asn Lys Thr Ile Ile Phe Lys Gln Ser
ATA GCT AGC AAA TTA AGA GAA CAA TTT GGA AAT AAT AAA ACA ATA ATC TTT AAG CAA TCC
7330

370
Ser Gly Gly Asp Pro Glu Ile Val Thr His Ser Phe Asn Cys Gly Gly Glu Phe Phe Tyr
TCA GGA GGG GAC CCA GAA ATT GTA ACG CAC AGT TTT AAT TGT GGA GGG GAA TTT TTC TAC
7390

390
Cys Asn Ser Thr Gln Leu Phe Asn Ser Thr Trp Phe Asn Ser Thr Trp Ser Thr Glu Gly
TGT AAT TCA ACA CAA CTG TTT AAT AGT ACT TGG TTT AAT AGT ACT TGG AGT ACT GAA GGG
7450

410
Ser Asn Asn Thr Glu Gly Ser Asp Thr Ile Thr Leu Pro Cys Arg Ile Lys Gln Phe Ile
TCA AAT AAC ACT GAA GGA AGT GAC ACA ATC ACA CTC CCA TGC AGA ATA AAA CAA TTT ATA
7510

430
Asn Met Trp Gln Glu Val Gly Lys Ala Met Tyr Ala Pro Pro Ile Ser Gly Gln Ile Arg
AAC ATG TGG CAG GAA GTA GGA AAA GCA ATG TAT GCC CCT CCC ATC AGC GGA CAA ATT AGA
7570

450
Cys Ser Ser Asn Ile Thr Gly Leu Leu Leu Thr Arg Asp Gly Gly Asn Asn Asn Asn Gly
TGT TCA TCA AAT ATT ACA GGG CTG CTA TTA ACA AGA GAT GGT GGT AAT AAC AAC AAT GGG
7630

470
Ser Glu Ile Phe Arg Pro Gly Gly Gly Asp Met Arg Asp Asn Trp Arg Ser Glu Leu Tyr
TCC GAG ATC TTC AGA CCT GGA GGA GGA GAT ATG AGG GAC AAT TGG AGA AGT GAA TTA TAT
     BglII                                           7690

490
Lys Tyr Lys Val Val Lys Ile Glu Pro Leu Gly Val Ala Pro Thr Lys Ala Lys Arg Arg
AAA TAT AAA GTA GTA AAA ATT GAA CCA TTA GGA GTA GCA CCC ACC AAG GCA AAG AGA AGA
7750

510. Extracellar Region-------\ /-------- Transmembrane Region
Val Val Gln Arg Glu Lys Arg Ala Val Gly Ile Gly Ala Leu Phe Leu Gly Phe Leu Gly
GTG GTG CAG AGA GAA AAA AGA GCA GTG GGA ATA GGA GCT TTG TTC CTT GGG TTC TTG GGA
7810

0 265 785

FIG. 1

530
Ala Ala Gly Ser Thr Met Gly Ala Arg Ser Met Thr Leu Thr Val Gln Ala Arg Gln Leu
GCA GCA GGA AGC ACT ATG GGC GCA CGG TCA ATG ACG CTG ACG GTA CAG GCC AGA CAA TTA
                                                                              7870
550
Leu Ser Gly Ile Val Gln Gln Gln Asn Asn Leu Leu Arg Ala Ile Glu Ala Gln Gln His
TTG TCT GGT ATA GTG CAG CAG CAG AAC AAT TTG CTG AGG GCT ATT GAG GCG CAA CAG CAT
                                                                              7930
570
Leu Leu Gln Leu Thr Val Trp Gly Ile Lys Gln Leu Gln Ala Arg Ile Leu Ala Val Glu
CTG TTG CAA CTC ACA GTC TGG GGC ATC AAG CAG CTC CAG GCA AGA ATC CTG GCT GTG GAA
                                                                              7990
590
Arg Tyr Leu Lys Asp Gln Gln Leu Leu Gly Ile Trp Gly Cys Ser Gly Lys Leu Ile Cys
AGA TAC CTA AAG GAT CAA CAG CTC CTG GGG ATT TGG GGT TGC TCT GGA AAA CTC ATT TGC
                                                                              8050
610
Thr Thr Ala Val Pro Trp Asn Ala Ser Trp Ser Asn Lys Ser Leu Glu Gln Ile Trp Asn
ACC ACT GCT GTG CCT TGG AAT GCT AGT TGG AGT AAT AAA TCT CTG GAA CAG ATT TGG AAT
                                                                              8110
Asn Met Thr Trp Met Glu Trp Asp Arg Glu Ile Asn Asn Tyr Thr Ser Leu Ile His Ser
AAC ATG ACC TGG ATG GAG TGG GAC AGA GAA ATT AAC AAT TAC ACA AGC TTA ATA CAT TCC
                                                                HindIII        8170
650
Leu Ile Glu Glu Ser Gln Asn Gln Gln Glu Lys Asn Glu Gln Glu Leu Leu Glu Leu Asp
TTA ATT GAA GAA TCG CAA AAC CAG CAA GAA AAG AAT GAA CAA GAA TTA TTG GAA TTA GAT
                                                                              8230
670
Lys Trp Ala Ser Leu Trp Asn Trp Phe Asn Ile Thr Asn Trp Leu Trp Tyr Ile Lys Ile
AAA TGG GCA AGT TTG TGG AAT TGG TTT AAC ATA ACA AAT TGG CTG TGG TAT ATA AAA ATA
                                                                              8290
690
Phe Ile Met Ile Val Gly Gly Leu Val Gly Leu Arg Ile Val Phe Ala Val Leu Ser Ile
TTC ATA ATG ATA GTA GGA GGC TTG GTA GGT TTA AGA ATA GTT TTT GCT GTA CTT TCT ATA
                                                                              8350
710
Val Asn Arg Val Arg Gln Gly Tyr Ser Pro Leu Ser Phe Gln Thr His Leu Pro Thr Pro
GTG AAT AGA GTT AGG CAG GGA TAT TCA CCA TTA TCG TTT CAG ACC CAC CTC CCA ACC CCG
                                                                              8410

0 265 785

FIG. 1

730
Arg Gly Pro Asp Arg Pro Glu Gly Ile Glu Glu Glu Gly Gly Glu Arg Asp Arg Asp Arg
AGG GGA CCC GAC AGG CCC GAA GGA ATA GAA GAA GAA GGT GGA GAG AGA GAC AGA GAC AGA
8470

750
Ser Ile Arg Leu Val Asn Gly Ser Leu Ala Leu Ile Trp Asp Asp Leu Arg Ser Leu Cys
TCC ATT CGA TTA GTG AAC GGA TCC TTA GCA CTT ATC TGG GAC GAT CTG CGG AGC CTG TGC
BamHI                                                       8530

770
Leu Phe Ser Tyr His Arg Leu Arg Asp Leu Leu Leu Ile Val Thr Arg Ile Val Glu Leu
CTC TTC AGC TAC CAC CGC TTG AGA GAC TTA CTC TTG ATT GTA ACG AGG ATT GTG GAA CTT
8590

790
Leu Gly Arg Arg Gly Trp Glu Ala Leu Lys Tyr Trp Trp Asn Leu Leu Gln Tyr Trp Ser
CTG GGA CGC AGG GGG TGG GAA GCC CTC AAA TAT TGG TGG AAT CTC CTA CAG TAT TGG AGT
8650

810
Gln Glu Leu Lys Asn Ser Ala Val Ser Leu Leu Asn Ala Thr Ala Ile Ala Val Ala Glu
CAG GAA CTA AAG AAT AGT GCT GTT AGC TTG CTC AAT GCC ACA GCC ATA GCA GTA GCT GAG
8710

830
Gly Thr Asp Arg Val Ile Glu Val Val Gln Gly Ala Cys Arg Ala Ile Arg His Ile Pro
GGG ACA GAT AGG GTT ATA GAA GTA GTA CAA GGA GCT TGT AGA GCT ATT CGC CAC ATA CCT
8770

851                                              861
Arg Arg Ile Arg Gln Gly Leu Glu Arg Ile Leu Leu End
AGA AGA ATA AGA CAG GGC TTG GAA AGG ATT TTG CTA TAA GATGGGTGGCAAGTGGTCAAAAAGTAGT
8837

GTGGTTGGATGGCCTACTGTAAGGGAAAGAATAGCGAGCTGACCAGCAGCAGATGGGGTGGGAGCAGCATCTCGAGACCT
8917

GGAAAAACATGGAGCAATCACAAGTAGCAATACAGCAGCTACCAATGCTGCTTGTGCCTGGCTAGAAGCACAAGAGGAGG
8997

AGGAGGTGGGTTTTCCAGTCACACCTCAGGTACCTTTAAGACCAATGACTTACAAGGCAGCTGTAGATCTTAGCCACTTT
Kpn                                                      Bgl II
9077

TTAAAAGAAAAGGGGGGACTG
9100

0 265 785

FIG. 2A

Synthetic Oligo

Gly Arg⊕ Val Lys⊕ Glu⊖ Lys⊕ Tyr Gln His Leu Trp Arg⊕ Trp Gly Trp Lys⊕ Trp Gly Thr Met Leu
CCC GGG CGT GTG AAG GAG AAG TAC CAA CAC CTG TGG CGT TGG GGC TGG AAG TGG GGC ACC ATG CTG
Leu Gly Ile Leu Met Ile Cys Ser Ala Thr Glu Lys⊕ Leu Trp Val Thr Val Tyr Tyr
CTG GGC ATC CTG ATG ATC TGT AGC GCT ACC GAG AAG CTG TGG GTG ACC GTG TAC TAC

SmaI

Gly Val Pro Val
GGG GTA CCT GTG ....

406

Hind/Sph/Pst/Sal/Xba/Bam/Sma

KpnI
127
DraI   BglII   580   BglII   HindIII   KpnI/Sst/EcoRI

BamHI

0        127    387    755        1335   1933   2810
                                          2250

pUC19

XmnI
(−1000)

PvuI
3840

PvuI
2944

Plasmid was constructed in 2 stages
1) Kpn-Kpn 2700 bp fragment of LAV ENV to Kpn site of pUC18 = p1614
2) 121 bp oligomer was blunt-end ligated to SmaI site

FIG. 2B

pUC8

BstEII 0.96

SstII 1.08

PvuII 1.35

XhoI 1.90

SalI 2.87

SalI 3.18

EcoRI 6.70

PvuII 6.20

HindIII 5.95

SalI 5.65

BamHI 5.56

pMGS3

Ap$^R$

polyhedrin

EcoRV 3.90

4.00

HindIII 4.63

SmaI/KpnI/BglII/STOP's

A₇CCTATAAATAATG CCC GGG GGT ACC AGA TCT TAA TTA ATT AAG T - 3'
polyhedrin promoter

SmaI    KpnI    BglII    STOP's

pUC8

BstEII 0.96

SstII 1.08

PvuII 1.35

XhoI 1.90

SalI 2.87

SalI 3.18

EcoRI 6.70

PvuII 6.20

HindIII 5.95

SalI 5.65

BamHI 5.56

pMGS1

Ap$^R$

polyhedrin

EcoRV 3.90

SmaI/KpnI 4.00

HindIII 4.63

5'- polyhedrin   ATG CCC GGG GGT ACC GAC TCT-3'
promoter

SmaI    KpnI
XmaI

pUC8

BstEII 0.96

SstII 1.08

PvuII 1.35

XhoI 1.90

SalI 2.87

SalI 3.18

EcoRI 6.70

PvuII 6.20

HindIII 5.95

SalI 5.65

BamHI 5.56

pMGS4

Ap$^R$

polyhedrin

EcoRV 3.90

4.00

HindIII 4.63

10aa poly/SmaI/KpnI/BglII/STOP's

met pro asp⊖ tyr ser tyr arg⊕ pro thr ile gly pro
TAATG CCC GAT TAT TCA TAC CGT CCC ACC ATC GGG CCC GGG GGT ACC AGA TCT TAA TTA ATT AAG T - 3'

SmaI    KpnI  BglII    STOP's

# FIG. 3A

FIG. 3B

pUC8

EcoRI
6.70

BstEII
0.96

SstII
1.08

PvuII
1.35

XhoI
1.90

ApR

pMGS3 +2

PvuII
6.20

HindIII
5.95

SalI
5.65

BamHI
5.56

SalI
2.87

polyhedrin

SalI
3.18

EcoRV
3.90

4.00

HindIII
4.63

SmaI/KpnI/BglII/STOP's

5'-polyhedrin- A₇CCTATAAATAATG CCC CCG GGG TAC CAG ATC TTA ATT AAT TAA GT -3'
promoter

SmaI    KpnI BglII    STOP's

0 265 785

FIG. 3C

pUC8

0

BstEⅡ
0.96

SstⅡ
1.08

PvuⅡ
1.35

XhoI
1.90

ApR

pMGS5

polyhedrin

6.89

PvuⅡ
6.39

HindⅢ
6.14

SalI
5.84

BamHI
5.75

SalI
2.87

SalI
3.18

EcoRⅤ
3.90

4.00

HindⅢ
4.81

SmaI/IL2signal/EcoRI/KpnI/BglⅡ/STOP's

met pro gly tyr arg met gln leu leu ser cys ile ala
5'- polyhedrin- A₇CCTATAAATAATG CCC GGG TAC CGT ATG CAA CTG CTG AGC TGC ATC GCT
promoter                          SmaI

leu ser leu ala leu val thr asn ser ala asn ser val pro asp leu asn STOP
CTG AGC CTG GCT CTG GTT ACC AAC AGC GCG AAT TCG GTA CCA GAT CCA GAT CTT AAT TAA TTA - 3'
                                    EcoRI              KpnI
                                                        BglⅡ
                            Cleavage site

No sites for: PstI, NcoI, XbaI, or SstI

FIG. 4

FIG. 5